(19) Europäisches Patentamt
European Patent Office
Office européen des brevets

(11) Veröffentlichungsnummer: **0 135 186**
**A2**

(12) # EUROPÄISCHE PATENTANMELDUNG

(21) Anmeldenummer: **84110760.0**

(22) Anmeldetag: **10.09.84**

(51) Int. Cl.⁴: **C 07 D 285/34**
**C 07 D 417/12, A 01 N 43/88**

(30) Priorität: **17.09.83 DE 3333659**

(43) Veröffentlichungstag der Anmeldung:
**27.03.85 Patentblatt 85/13**

(84) Benannte Vertragsstaaten:
**AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Brandes, Wilhelm, Dr.**
**Eichendorffstrasse 3**
**D-5653 Leichlingen 1(DE)**

(72) Erfinder: **Bunnenberg, Rolf, Dr.**
**Neuenkamperweg 12**
**D-5653 Leichlingen 1(DE)**

(72) Erfinder: **Hänssler, Gerd, Dr.**
**Heymannstrasse 40**
**D-5090 Leverkusen 1(DE)**

(72) Erfinder: **Reinecke, Paul, Dr.**
**Lessingstrasse 11**
**D-5090 Leverkusen 3(DE)**

(72) Erfinder: **Wedemeyer, Karlfried, Dr.**
**Bilharzstrasse 7**
**D-5000 Köln 80(DE)**

(54) **3,6-Disubstituierte 1,3,5-Thiadiazin-2-thioxo-4-one.**

(57) 3,6-Disubstituierte 1,3,5-Thiadiazin-2-thioxo-4-one der allgemeinen Formel (I)

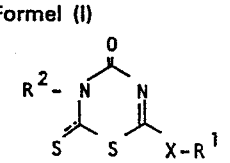

(I)

in welcher

$R^1$ für Wasserstoff, für gegebenenfalls einfach bei mehrfach, gleich oder verschieden substituiertes Alkyl, für gegebenenfalls einfach bis mehrfach, gleich oder verschieden substituiertes Alkenyl, für gegebenenfalls einfach bis mehrfach substituiertes Alkinyl, für gegebenenfalls einfach bis mehrfach, gleich oder verschieden substituiertes Cycloalkyl oder Cycloalkenyl, für gegebenenfalls einfach bis mehrfach, gleich oder verschieden substituiertes Aralkyl oder Aryl oder für einen einfach oder mehrfach, gleich oder verschieden substituierten Heterocyclus steht,

$R^2$ für gegebenenfalls einfach bis mehrfach, gleich oder verschieden substituiertes Alkyl oder Alkenyl; für gegebenenfalls einfach bis mehrfach, gleich oder verschieden substituiertes Cycloalkyl oder Cycloalkenyl, für einfach bis mehrfach, gleich oder verschieden substituiertes Aralkyl oder den Fluorenylrest steht und

X für Sauerstoff oder SChwefel steht, sowie ihre Verwendung als Schädlingsbekämpfungsmittel, vor allem als Fungizide.

Die neuen 3,6-disubstituierten 1,3,5-Thiadiazin-2-thioxo-4-one können hergestellt werden, wenn man geeignete 6-substituierte 1,3,5-Thiadiazin-2-thioxo-4-one mit geeigneten Diazoverbindungen umsetzt.

EP 0 135 186 A2

BAYER AKTIENGESELLSCHAFT 5090 Leverkusen, Bayerwerk

Konzernverwaltung RP
Patentabteilung Bas/Hed-c

Ia

## 3,6-Disubstituierte 1,3,5-Thiadiazin-2-thioxo-4-one

Die vorliegende Erfindung betrifft neue 3,6-disubstituierte 1,3,5-Thiadiazin-2-thioxo-4-one, ein Verfahren zu ihrer Herstellung und ihre Verwendung als Schädlingsbekämpfungsmittel.

Es ist bereits bekannt geworden, daß monosubstituierte 1,3,5-Thiadiazine, wie z.B. das 2,3-Dihydro-6-methoxy-2-thioxo-4H-1,3,5-thiadiazin-4-on, gute fungizide Eigenschaften aufweisen (vgl. DE-OS 3 010 238).

Weiterhin sind di- und trisubstituierte 1,3,5-Thiadiazinthioxo-Verbindungen bekannt, wie z.B. das 3,5-Dimethyl-tetrahydro-1,3,5-thiadiazinthion-(2), das 3-Methyl-5-carboxymethyl-tetrahydro-1,3,5-thiadiazinthion-(2) und das 3,3'-Ethylen-bis-/tetrahydro-4,6-dimethyl-1,3,5-thiadiazinthion-(2)/. Auch diese Verbindungen weisen fungizide Eigenschaften auf (vgl. z.B. R. Wegler, "Chemie der Pflanzenschutz- und Schädlingsbekämpfungsmittel", Bd. 2, Seiten 126- 127, Springer Verlag Berlin-Heidelberg-New York 1970).

Bei all diesen Verbindungen ist unter bestimmten Umständen, z.B. bei niedrigen Anwendungskonzentrationen, die Wirkung nicht immer voll befriedrigend.

Es wurden neue 3,6-disubstituierte 1,3,5-Thiadiazin-2-thioxo-4-one der allgemeinen Formel (I)

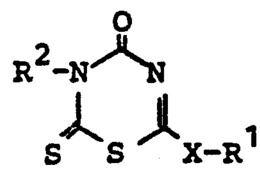

(I)

in welcher

$R^1$ für Wasserstoff; für gegebenenfalls einfach bis mehrfach, gleich oder verschieden substituiertes Alkyl; für gegebenenfalls einfach bis mehrfach, gleich oder verschieden substituiertes Alkenyl; für gegebenenfalls einfach bis mehrfach substituiertes Alkinyl; für gegebenenfalls einfach bis mehrfach, gleich oder verschieden substituiertes Cycloalkyl oder Cycloalkenyl; für gegebenenfalls einfach bis mehrfach, gleich oder verschieden substituiertes Aralkyl oder Aryl oder für einen einfach oder mehrfach, gleich oder verschieden substituierten Heterocyclus steht,

$R^2$ für gegebenenfalls einfach bis mehrfach, gleich oder verschieden substituiertes Alkyl oder Alkenyl; für gegebenenfalls einfach bis mehrfach, gleich oder verschieden substituiertes Cycloalkyl oder Cycloalkenyl; für ggf. einfach bis mehrfach, gleich oder verschieden substituiertes Aralkyl oder den Fluorenylrest steht und

X für Sauerstoff oder Schwefel steht,

gefunden.

Weiterhin wurde gefunden, daß man die 3,6-disubstituierten 1,3,5-Thiadiazin-2-thioxo-4-one der Formel (I)

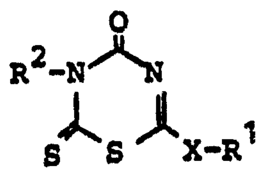

(I)

in welcher

$R^1$ für Wasserstoff; für gegebenenfalls einfach bis mehrfach, gleich oder verschieden substituiertes Alkyl; für gegebenenfalls einfach bis mehrfach, gleich oder verschieden substituiertes Alkenyl; für gegebenenfalls einfach bis mehrfach substituiertes Alkinyl; für gegebenenfalls einfach bis mehrfach, gleich oder verschieden substituiertes Cycloalkyl oder Cycloalkenyl; für gegebenenfalls einfach bis mehrfach, gleich oder verschieden substituiertes Aralkyl oder Aryl oder für einen einfach oder mehrfach, gleich oder verschieden substituierten Heterocyclus steht,

$R^2$ für gegebenenfalls einfach bis mehrfach, gleich oder verschieden substituiertes Alkyl oder Alkenyl; für gegebenenfalls einfach bis mehrfach, gleich oder verschieden substituiertes Cycloalkyl oder Cycloalkenyl; für einfach bis mehrfach, gleich oder verschieden substituiertes Aralkyl oder den Fluorenylrest steht und

X für Sauerstoff oder Schwefel steht, erhält,

wenn

man 1,3,5-Thiadiazine der Formel (II)

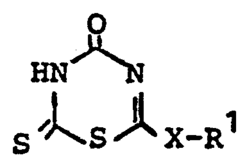

(II)

in welcher

$R^1$ und X die oben angegebene Bedeutung haben,

mit Diazoverbindungen der Formel (III)

$$R^{2'} = N_2 \qquad \text{(III)}$$

in welcher

$R^{2'}$ für gegebenenfalls ein- bis mehrfach, gleich oder verschieden substituiertes Alkylen, Alkenylen, Cycloalkylen, Cycloalkenylen, Aralkylen oder den Fluorenylenrest steht, gegebenenfalls in Gegenwart eines inerten Verdünnungsmittels umsetzt.

Die neuen 3,6-disubstituierten 1,3,5-Thiadiazin-2-thioxo-4-one der Formel (I) weisen starke biologische, vor allem fungizide Eigenschaften auf. Dabei zeigen überraschenderweise die erfindungsgemäßen Verbindungen eine bessere fungizide Wirkung, als die aus dem Stand der Technik bekannten fungiziden Wirkstoffe.

Die neuen Verbindungen stellen somit eine Bereicherung der Technik dar.

Die erfindungsgemäßen 3,6-disubstituierten 1,3,5-Thiadiazin-2-thioxo-4-one sind durch die Formel (I) allgemein definiert. Von den neuen Verbindungen der Formel (I) sind bevorzugt diejenigen in denen

$R^1$ für Wasserstoff; für ein- bis fünffach, gleich oder verschieden substituiertes Alkyl mit 1 bis 12 Kohlenstoffatomen, wie z.B. Methyl, Ethyl, Propyl, Isopropyl, Butyl, Isobutyl, tert.-Butyl, Pentyl, Hexyl, Isohexyl, Octyl, Isooctyl, Decyl, Isodecyl, Dodecyl oder Isododecyl, steht, wobei als Substituenten Nitro, Cyano, Isocyanato, Halogen, Hydroxy,

Alkoxy mit 1 bis 4 Kohlenstoffatomen, Mercapto, Alkylthio mit 1 bis 4 Kohlenstoffatomen, Alkylcarbonyl mit 1 bis 5 Kohlenstoffatomen im Alkylteil, Alkylcarbonyloxy mit 1 bis 5 Kohlenstoffatomen und Alkoxycarbonyl mit 1 bis 5 Kohlenstoffatomen im Alkyloxyteil genannt seien; für gegebenenfalls ein- bis fünffach, gleich oder verschieden substituiertes Alkenyl mit 2 bis 12 Kohlenstoffatomen, wie z.B. Allyl, Butenyl, Isobutenyl, Hexenyl, Isohexenyl, Octenyl, Isooctenyl, Decenyl, Isodecenyl, Dodecenyl und Isododecenyl steht, wobei als Substituenten Halogen, Cyano, Alkoxy mit 1 bis 4 Kohlenstoffatomen, Alkylthio mit 1 bis 4 Kohlenstoffatomen, Alkylcarbonyl und Alkylcarbonyloxy mit je 1 bis 5 Kohlenstoffatomen im Alkylteil und Alkoxycarbonyl mit 1 bis 5 Kohlenstoffatomen genannt seien; für gegebenenfalls ein- bis fünffach substituiertes Alkinyl mit 3 bis 12 Kohlenstoffatomen, wie beispielsweise Propargyl, Butinyl, Pentinyl, Hexinyl, Isohexinyl, Heptinyl, Octinyl und Dodecinyl steht, wobei als Substituenten Halogen, Alkoxy und Alkylthio mit 1 bis 4 Kohlenstoffatomen je Alkylteil, Alkylcarbonyl und Alkylcarbonyloxy mit 1 bis 5 Kohlenstoffatomen je Alkylteil und Alkoxycarbonyl mit 1 bis 5 Kohlenstoffatomen genannt seien; für gegebenenfalls ein- bis fünffach, gleich oder verschieden substituiertes Cycloalkyl mit 3 bis 9 Kohlenstoffatomen, wie beispielsweise Cyclopropyl, Cyclobutyl, Cyclopentyl, Cyclohexyl, Cycloheptyl oder Cyclooctyl, steht, wobei als Substituenten Halogen, Alkoxy und Alkylthio mit je 1 bis 4

Kohlenstoffatomen, Alkyl, Alkylcarbonyl und Alkylcarbonyloxy mit 1 bis 5 Kohlenstoffatomen je Alkylteil, Alkoxycarbonyl mit 1 bis 5 Kohlenstoffatomen im Alkoxyteil und Halogenalkyl mit 1 bis 3 Kohlenstoffatomen und 1 bis 5 gleichen oder verschiedenen Halogenatomen genannt seien; für gegebenenfalls ein- bis fünffach gleich oder verschieden substituiertes Cycloalkenyl mit 3 bis 8 Kohlenstoffatomen, wie beispielsweise Cyclopentenyl, Cyclohexenyl, Cycloheptenyl oder Cyclooctenyl, steht, wobei als Substituenten Halogen, Alkyl und Alkoxy mit je 1 bis 4 Kohlenstoffatomen und Halogenalkyl mit 1 bis 3 Kohlenstoffatomen und 1 bis 5 gleichen oder verschiedenen Halogenatomen genannt seien; für gegebenenfalls ein- bis fünffach gleich oder verschieden substituiertes Aryl mit 6 bis 14 Kohlenstoffatomen oder Aralkyl mit 1 bis 3 Kohlenstoffatomen im Alkylteil und 6 bis 14 Kohlenstoffatomen im Arylteil, wie z.B. Phenyl, Naphthyl, Anthryl oder Benzyl, α-Phenylethyl, ß-Phenylethyl, Naphthylmethyl, Naphthylethyl, Anthrylmethyl und Anthrylethyl, steht, wobei als Substituenten im Arylteil Halogen, Phenyl, Nitro, Cyano, Isocyanato, Hydroxy, Mercapto, Alkyl mit 1 bis 4 Kohlenstoffatomen, Alkoxy und Alkoxycarbonyl mit 1 bis 5 Kohlenstoffatomen je Alkoxyteil, Alkylcarbonyl und Alkylcarbonyloxy mit je 1 bis 5 Kohlenstoffatomen je Alkylteil, Alkylthio mit 1 bis 5 Kohlenstoffatomen, Halogenalkyl, Halogenalkoxy und Halogenalkylthio mit 1 bis 3 Kohlenstoffatomen und 1 bis 5 gleichen oder verschiedenen Halogenatomen je Halogenalkylrest; genannt seien; für gegebenenfalls einfach bis fünffach, gleich oder

verschieden substituierten 5- oder 6-gliedrigen Heterocyclus mit 1 bis 3 gleichen oder verschiedenen Heteroatomen, wie Sauerstoff, Schwefel oder Stickstoff, steht, wobei als Substituenten Halogen, Alkyl mit 1 bis 4 Kohlenstoffatomen, Halogenalkyl und Halogenalkoxy mit 1 bis 3 Kohlenstoffatomen und 1 bis 5 gleichen oder verschiedenen Halogenatomen, Alkoxy mit 1 bis 6 Kohlenstoffatomen oder 2 Substituenten an einem Kohlenstoffatom gemeinsam einen Alkylenrest mit 4 oder 5 Kohlenstoffatomen bilden können, genannt seien;

$R^2$ für ein- bis fünffach, gleich oder verschieden substituiertes Alkyl oder Alkenyl mit bis zu 12 Kohlenstoffatomen wie unter $R^1$ angegeben steht, wobei als Substituenten Halogen, Nitro, Cyano oder Alkoxy mit 1 bis 5 Kohlenstoffatomen genannt seien; für ein- bis fünffach, gleich oder verschieden substituiertes Cycloalkyl oder Cycloalkenyl je mit 3 bis 8 Kohlenstoffatomen wie unter $R^1$ angegeben steht, wobei als Substituenten Halogen, Alkyl und Alkoxy mit je 1 bis 4 Kohlenstoffatomen und Halogenalkyl mit 1 bis 3 Kohlenstoffatomen und 1 bis 5 gleichen oder verschiedenen Halogenatomen genannt seien; ferner für gegebenenfalls ein- bis fünffach, gleich oder verschieden substituierten Fluorenylrest oder Aralkyl mit 6 bis 14 Kohlenstoffatomen im Arylteil und 1 bis 3 Kohlenstoffatomen im Alkylteil, wie z.B. Benzyl, steht, wobei als Substituenten im Arylteil Phenyl, Halogen, Nitro, Cyano, Alkyl oder Alkoxy mit je 1 bis 5 Kohlenstoffatomen genannt seien und

X für Sauerstoff oder Schwefel steht.

Ganz besonders bevorzugt sind diejenigen Verbindungen der Formel (I), in denen

$R^1$ für Wasserstoff; für geradkettiges oder verzweigtes Alkyl mit 1 bis 12 Kohlenstoffatomen; für Alkenyl mit 2 bis 6 Kohlenstoffatomen; für einfach durch Nitro, Cyano, Isocyanato, Fluor, Chlor, Brom, Jod, Hydroxy, Alkoxy mit 1 bis 3 Kohlenstoffatomen, wie Methoxy, Ethoxy, n- und iso-Propoxy, Mercapto, Alkylthio mit 1 bis 3 Kohlenstoffatomen, wie Methylthio, Ethylthio, n- und iso-Propylthio, Alkylcarbonyl und Alkylcarbonyloxy mit 1 bis 3 Kohlenstoffatomen im Alkylteil, wie Methylcarbonyl, Methylcarbonyloxy, Ethylcarbonyl, Ethylcarbonyloxy, n- und iso-Propylcarbonyl und n- und iso-Propylcarbonyloxy, und Alkoxycarbonyl mit 1 bis 3 Kohlenstoffatomen im Alkoxyteil, wie Methoxycarbonyl, Ethoxycarbonyl, n- und iso-Propoxycarbonyl substituiertes Alkyl mit 1 bis 4 Kohlenstoffatomen und Alkenyl mit 2 bis 6 Kohlenstoffatomen; für einfach durch Fluor, Chlor, Brom, Jod, Alkoxy oder Alkylthio mit je 1 bis 3 Kohlenstoffatomen, wie Methoxy, Ethoxy, n- und iso-Propoxy, Methylthio, Ethylthio, n- und iso-Propylthio, Alkylcarbonyl und Alkylcarbonyloxy mit je 1 bis 3 Kohlenstoffatomen je Alkylteil, wie Methylcarbonyl, Ethylcarbonyl, Propylcarbonyl, Methylcarbonyloxy, Ethylcarbonyloxy und Propylcarbonyloxy, substituiertes Alkinyl mit 3 oder 4 Kohlenstoffatomen im Alkinylteil; für gegebenenfalls ein- bis

dreifach durch Methyl oder Ethyl substituiertes Cyclopentyl, Cyclohexyl, Cyclopentenyl, Cyclohexenyl, und Cycloheptenyl; für gegebenenfalls ein- bis dreifach, gleich oder verschieden durch Fluor, Chlor, Brom, Jod, Nitro, Cyano, Isocyanato, Phenyl, Methyl, Ethyl, n- und iso-Propyl, tert.-Butyl, Mercapto, Methoxy, Ethoxy, Propoxy, Methoxycarbonyl, Ethoxycarbonyl, Methylcarbonyl, Ethylcarbonyl, Propylcarbonyl, Trichlormethyl, Flurodichlormethyl, Trichlorethyl, Trichlormethoxy und Trichlorethoxy substituiertes Phenyl, Naphthyl, Benzyl, Phenylethyl, Naphthylethyl und Naphthylmethyl; für gegebenenfalls ein- bis dreifach durch Fluor, Chlor, Methyl, Ethyl oder Propyl substituiertes 1,3-Dioxa-cyclohexyl, Furyl, Pyrrolyl, Imidazolyl, Pyrazinyl, Isoxazolyl, Morpholinyl und 1,5-Dioxaspiroundecanyl steht;

$R^2$ für Alkyl mit 1 bis 5 Kohlenstoffatomen; für einfach durch Fluor, Chlor, Brom, Cyano, Alkoxy mit 1 bis 3 Kohlenstoffatomen, wie Methoxy, Ethoxy und Propoxy, substituiertes Alkyl und Alkenyl mit bis zu 6 Kohlenstoffatomen je Rest; für gegebenenfalls durch Alkyl mit 1 bis 3 Kohlenstoffatomen, wie Methyl, Ethyl und Propyl, substituiertes Cycloalkyl und Cycloalkenyl mit 4 bis 7 Kohlenstoffatomen und für gegebenenfalls ein- bis dreifach im Arylteil durch Phenyl, Fluor, Chlor, Brom, Nitro, Alkyl und Alkoxy mit je 1 bis 3 Kohlenstoffatomen, wie Methyl, Ethyl, Propyl, Methoxy, Ethoxy und Propoxy, substituiertes Benzyl und Fluorenyl steht und

X für Sauerstoff oder Schwefel steht.

Verwendet man beispielsweise 2,3-Dihydro-6-phenylthio-2-thioxo-4H-1,3,5-thiadiazin-4-on und Diazomethan, so kann der Reaktionsablauf durch das folgende Formelschema wiedergegeben werden:

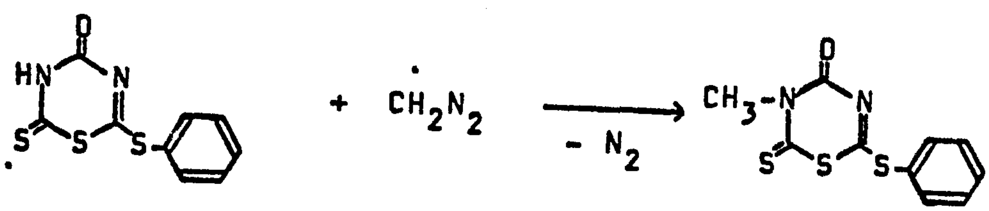

Die bei der Durchführung des erfindungsgemäßen Verfahrens als Ausgangsstoffe zu verwendenden 1,3,5-Thiadiazin-2-thioxo-4-one sind durch die Formel (II) allgemein definiert. In dieser Formel stehen $R^1$ und X vorzugsweise für diejenigen Reste, die bereits im Zusammenhang mit der Beschreibung der erfindungsgemäßen Stoffe der Formel (I) vorzugsweise für diese Substituenten genannt wurden.

Die 1,3,5-Thiadiazine der Formel (II) sind teilweise bekannt (vgl. DE-OS 3 010 204, DE-OS 2 010 237 und DE-OS 3 010 238).

Sowohl die neuen als auch die bekannten Verbindungen können nach dem dort angegebenen Verfahren erhalten werden, indem man das Carbonyldiisothiocyanat der Formel (IV)

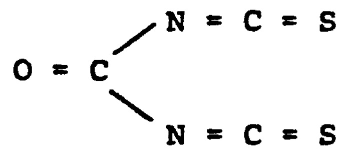

(IV)

mit Verbindungen der Formel (V)

$$R^1 - X - H \qquad (V)$$

in welcher

$R^1$ und X die oben angebene Bedeutung haben,

in Gegenwart eines inerten Lösungsmittels, wie beispielsweise Ether, Ester, Kohlenwasserstoffe oder halogenierte Kohlenwasserstoffe, umsetzt.

Die weiterhin als Ausgangsstoffe benötigten Diazoverbindungen der Formel (III) sind größtenteils bekannt oder können in allgemein bekannter Art und Weise erhalten werden (vgl. Houben-Weyl Bd. X/4).

Für die erfindungsgemäße Umsetzung kommen als Verdünnungsmittel inerte organische Lösungsmittel infrage. Hierzu gehören vorzugsweise Ether, wie beispielsweise Diethylether, Dimethoxyethan, Tetrahydrofuran, Dioxan; Kohlenwasserstoffe, wie beispielsweise Hexan, Ligroinfraktionen,

Benzol, Toluol, Xylol; halogenierte Kohlenwasserstoffe, wie beispielsweise Methylenchlorid, Chloroform, Tetrachlorkohlenstoff, Chlorbenzol oder Dichlorbenzol. Besonders bevorzugt wird einer der genannten Ether verwendet.

Die Reaktionstemperaturen können in einem größeren Bereich variiert werden. Im allgemeinen arbeitet man zwischen -10°C und +60°C, vorzugsweise bei 0°C bis 30°C.

Bei der Durchführung des erfindungsgemäßen Verfahrens setzt man auf 1 Mol der Verbindung der Formel (II) vorzugsweise 1 bis 1,5 Mol der Verbindung der Formel (III) ein. Zur Isolierung der Verbindungen der Formel (I) wird das Lösungsmittel abdestilliert und der Rückstand durch Umkristallisation oder Extraktion gereinigt. In einer Variante des erfindungsgemäßen Verfahrens wird das durch Reaktion des Carbonyldiisothiocyanats der Formel (IV) mit Verbindungen der Formel (V) gebildete 1,3,5-Thiadiazin der Formel (II) zwischenzeitlich nicht isoliert, sondern gleich mit einer Verbindung der Formel (III) zu 3,6-disubstituierten 1,3,5-Thiadiazin-2-thioxo-4-onen der allgemeinen Formel (I) umgesetzt.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

So können z.B. fungizide Mittel im Pflanzenschutz eingesetzt werden zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Im Pflanzenschutz können die erfindungsgemäßen Verbindungen mit besonders gutem Erfolg zur Bekämpfung von Puccinia-, Leptosphaeria- und Venturia-Arten, gegen pilzliche Krankheitserreger am Reis, wie z.B. gegen Pyricularia, eingesetzt werden. Außerdem weisen sie z.B. eine gute fungizide Wirkung gegen Mehltau, Cochliobolus sativus und Pyrenophora teres an Getreide auf. Bei entsprechender Anwendung zeigen sie auch insektizide und akarizide Wirksamkeit.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie

Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalo-

cyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinem zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei der Behandlung des Bodens sind Wirkstoffkonzentrationen von 0,00001 bis 0,1 Gew.-%, vorzugsweise von 0,0001 bis 0,02 %, am Wirkungsort erforderlich.

Das erfindungsgemäße Verfahren soll durch die folgenden Herstellungsbeispiele erläutert werden:

Herstellungsbeispiele

Beispiel 1

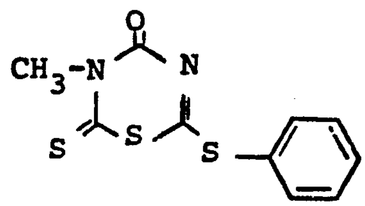

Zur Lösung von 10,2 g 2,3-Dihydro-6-phenylthio-2-thioxo-4H-1,3,5-thiadiazin-4-on in 200 ml Tetrahydrofuran gibt man bei 22°C portionsweise eine etherische Lösung von Diazomethan bis keine Stickstoffentwicklung mehr beobachtet wird. Überschüssiges Diazomethan wird durch Zugabe von Essigsäure zerstört, das Lösungsmittel abdestilliert, der Rückstand mit 20 ml Ether versetzt und der Niederschlag abgesaugt.

Man erhält 6,2 g 3-Methyl-6-phenylthio-2-thioxo-1,3,5-thiadiazin-4-on in Form gelber Kristalle vom Schmp. 116 - 118°C. Molmasse 268 (MS). IR (KBr): C=O = 1722 $cm^{-1}$, C=N 1548, C=S 1228
$^{1}H$-NMR ($CDCl_3$): $CH_3$ = 3,8 ppm (s), $CH_{arom.}$ 7,6 (m)
$C_{10}H_8N_2OS_3$ (268,4)

| | | | | |
|---|---|---|---|---|
| berechnet | C = 44,8 | H = 3,0 | N = 10,4 | S = 35,8 |
| gefunden | C = 44,3 | H = 3,0 | N = 10,4 | S = 36,2 |

Beispiel 2

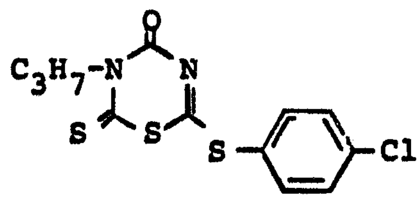

Die Lösung von 28,8 g 2,3-Dihydro-6-(4-chlorphenylthio)-2-thioxo-4H-1,3,5-thiadiazin-4-on in 250 ml Tetrahydrofuran wird bei 22°C portionsweise mit einer Lösung von 7,7 g Diazopropan in Ether versetzt. Das Lösungsmittel wird abdestilliert, der Rückstand in wenig Methanol aufgenommen, der Niederschlag abgesaugt, mit Methanol gewaschen und getrocknet. Es werden 21,9 g 3-Propyl-6-(4-chlorphenylthio)-2-thioxo-1,3,5-thiadiazin-4-on in Form gelber Kristalle vom Schmp. 151 - 152°C erhalten.

Herstellung des Ausgangsproduktes

Zur Lösung von 14,4 g Carbonyldiisothiocyanat in 150 ml Tetrahydrofuran tropft man bei 0 - 5°C eine Lösung von 14,4 g p-Chlorthiophenol in 150 ml Tetrahydrofuran. Man läßt 3 Stunden rühren und dabei auf 22°C aufwärmen. Durch Zugabe von Benzin werden 13,8 g gelbe Kristalle vom Schmp. 172-174°C (Zers.) ausgefällt.

In entsprechender Weise wie Beispiele 1 und 2 werden die nachfolgenden Beispiele der allgemeinen Formel (I)

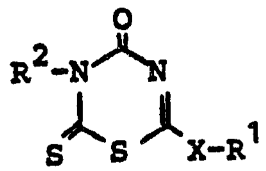

(I)

erhalten:

| Bsp. Nr. | $R^1$ | X | $R^2$ | Schmp. ( °C) |
|---|---|---|---|---|
| 3 | $-C_2H_5$ | O | $CH_3$ | 88 - 89 |
| 4 | $-C_2H_5$ | S | $CH_3$ | 75 - 76 |
| 5 | $-CH(CH_3)COOC_2H_5$ | O | $CH_3$ | 75 - 76 |
| 6 | $-CH_2-CH_2-O-CH_3$ | O | $CH_3$ | 61 - 62 |
| 7 | $-CH_2-$ 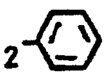 | O | $CH_3$ | 87 - 88 |
| 8 | 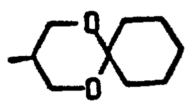 | O | $CH_3$ | Harz |
| 9 | $-C_{12}H_{25}$ | O | $CH_3$ | 57 - 58 |
| 10 | $-C_8H_{17}$ | O | $CH_3$ | 54 - 55 |
| 11 | $-CH_2-$ 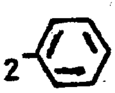 | S | $CH_3$ | 59 - 61 |
| 12 | - 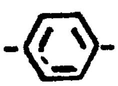 -Cl | S | $CH_3$ | 164 - 165 |
| 13 | $-C(CH_3)_3$ | S | $CH_3$ | 109 - 110 |
| 14 | $-CH_2-CH_2-OH$ | S | $CH_3$ | 135 - 136 |
| 15 | 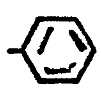 | S | $C_2H_5$ | 97 - 100 |
| 16 | $-CH_2-CH=CH_2$ | O | $CH_3$ | 45 - 46 |

| Bsp. Nr. | $R^1$ | X | $R^2$ | Schmp. (°C) |
|---|---|---|---|---|
| 17 | $-CH_3$ | S | $CH_3$ | 98 - 100 |
| 18 | $-C_{12}H_{25}$ | S | $CH_3$ | 55 - 56 |
| 19 | 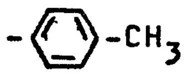 | S | $CH_3$ | 120 - 122 |
| 20 | 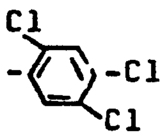 | S | $CH_3$ | 174 - 176 |
| 21 | 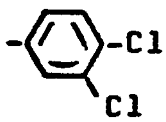 | S | $CH_3$ | 180 - 183 |
| 22 | 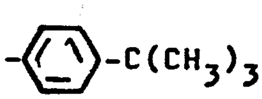 | S | $CH_3$ | 121 - 122 |
| 23 | $-CH_2-CH=CH_2$ | O | $C_2H_5$ | Öl |
| 24 | $-CH(CH_3)_2$ | O | $CH_3$ | 58-59 |
| 25 | $-CH_3$ | O | $CH_3$ | 91-92 |
| 26 | 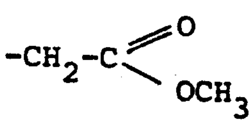 | S | $CH_3$ | 66-67 |
| 27 | 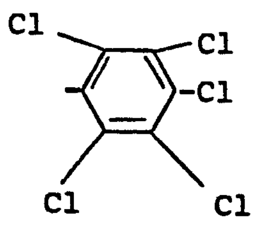 | S | $CH_3$ | 198-201 |

| Bsp. Nr. | $R^1$ | X | $R^2$ | Schmp. (°C) |
|---|---|---|---|---|
| 28 | 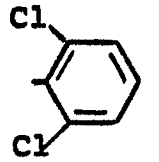 | S | $C_2H_5$ | 189-190 |
| 29 | 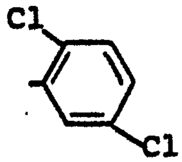 | S | $CH_3$ | 149-150 |
| 30 | 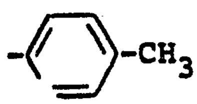 | S | $C_3H_7$-n | 117-119 |
| 31 | 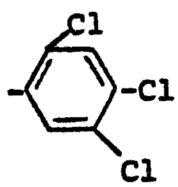 | S | $C_3H_7$-n | 145-149 |
| 32 | 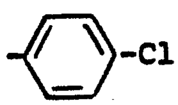 | S | $CH_3-O-CH_2-CH_2-$ | 116-118 |
| 33 | 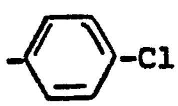 | S | $C_2H_5$ | 157-160 |
| 34 | 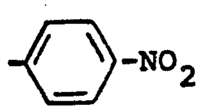 | S | $C_2H_5$ | 161-163 |
| 35 | 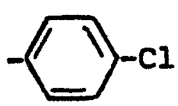 | S | $CH_3$ | 165-167 |
| 36 | 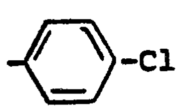 | S | $C_6H_5-CH_2-$ | Harz |
| 37 | 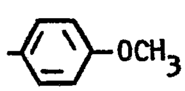 | S | $CH_3$ | 140-141 |
| 38 | $-CH_2-C\equiv CCl$ | O | $CH_3$ | 139-140 |

Anwendungsbeispiele

In den folgenden Anwendungsbeispielen wird die nachstehend aufgeführte Verbindung als Vergleichssubstanz eingesetzt:

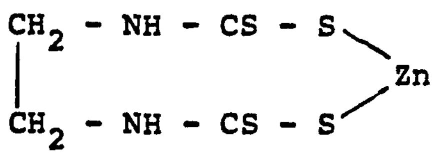

(A)

Zinkethylenbisdithiocarbamat

**Beispiel A**

Puccinia-Test (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita in einer 0,1 %igen wäßrigen Agarlösung inokuliert. Nach Antrocknen besprüht man die Pflanzen mit der Wirkstoffzubereitung taufeucht. Die Pflanzen verbleiben 24 Stunden bei 20°C und 100 % rel. Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt, um die Entwicklung von Rostpusteln zu begünstigen.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 1 und 5.

Beispiel B

Leptosphaeria nodorum-Test (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 15°C und einer relativen Luftfeuchtigkeit von ca. 80 % aufgestellt.

10 Tage nach der Inokulation erfolgt die Auswertung.

Eine deutliche Überlegenheit in der Wirksamkeit gegenüber dem Stand der Technik zeigt bei diesem Test z.B. die Verbindung gemäß Herstellungsbeispiel 1.

**Beispiel C**

Venturia-Test (Apfel) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100 % relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 1, 5 und 7.

Beispiel D

Pyricularia-Test (Reis) /protektiv

| | | | |
|---|---|---|---|
| Lösungsmittel: | 12,5 | Gewichtsteile | Aceton |
| Emulgator: | 0,3 | Gewichtsteile | Alkylarylpolyglykolether |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigt in diesem Test z.B. die Verbindung gemäß Herstellungsbeispiel 1.

**Beispiel E**

Pyricularia-Test (Reis) / systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß folgender Herstellungsbeispiele: 1 und 9.

Patentansprüche

1. 3,6-Disubstituierte 1,3,5-Thiadiazin-2-thioxo-4-one der allgemeinen Formel (I)

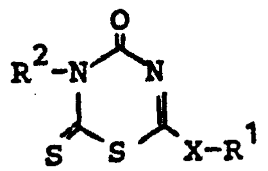

(I)

in welcher

$R^1$ für Wasserstoff; für gegebenenfalls einfach bis mehrfach, gleich oder verschieden substituiertes Alkyl; für gegebenenfalls einfach bis mehrfach, gleich oder verschieden substituiertes Alkenyl; für gegebenenfalls einfach oder mehrfach substituiertes Alkinyl; für gegebenenfalls einfach bis mehrfach, gleich oder verschieden substituiertes Cycloalkyl oder Cycloalkenyl; für gegebenenfalls einfach bis mehrfach, gleich oder verschieden substituiertes Aralkyl und Aryl oder für einen einfach oder mehrfach, gleich oder verschieden substituierten Heterocyclus steht,

$R^2$ für gegebenenfalls einfach bis mehrfach, gleich oder verschieden substituiertes Alkyl oder Alkenyl; für gegebenenfalls einfach bis mehrfach, gleich oder verschieden substituiertes Cycloalkyl oder Cycloalkenyl; für gegebenenfalls einfach bis mehrfach, gleich oder verschieden substituiertes Aralkyl oder den Fluorenylrest steht und

X für Sauerstoff oder Schwefel steht.

2. 3,6-Disubstituierte 1,3,5-Thiadiazin-2-thioxo-4-one der Formel (I) gemäß Anspruch 1, in welcher

$R^1$ für Wasserstoff; für ein- bis fünffach, gleich oder verschieden substituiertes Alkyl mit 1 bis 12 Kohlenstoffatomen steht, wobei als Substituenten Nitro, Cyano, Isocyanato, Halogen, Hydroxy, Alkoxy mit 1 bis 4 Kohlenstoffatomen, Mercapto, Alkylthio mit 1 bis 4 Kohlenstoffatomen, Alkylcarbonyl mit 1 bis 5 Kohlenstoffatomen im Alkylteil, Alkylcarbonyloxy mit 1 bis 5 Kohlenstoffatomen und Alkoxycarbonyl mit 1 bis 5 Kohlenstoffatomen im Alkyloxyteil genannt seien; für gegebenenfalls ein- bis fünffach, gleich oder verschieden substituiertes Alkenyl mit 2 bis 12 Kohlenstoffatomen steht, wobei als Substituenten Halogen, Cyano, Alkoxy mit 1 bis 4 Kohlenstoffatomen, Alkylthio mit 1 bis

4 Kohlenstoffatomen, Alkylcarbonyl und Alkylcarbonyloxy mit je 1 bis 5 Kohlenstoffatomen im Alkylteil und Alkoxycarbonyl mit 1 bis 5 Kohlenstoffatomen genannt seien; für gegebenenfalls ein- bis fünffach substituiertes Alkinyl mit 3 bis 12 Kohlenstoffatomen steht, wobei als Substituenten Halogen, Alkoxy und Alkylthio mit je 1 bis 4 Kohlenstoffatomen je Alkylteil, Alkylcarbonyl und Alkylcarbonyloxy mit 1 bis 5 Kohlenstoffatomen je Alkylteil und Alkoxycarbonyl mit 1 bis 5 Kohlenstoffatomen genannt seien; für gegebenenfalls ein- bis fünffach, gleich oder verschieden substituiertes Cycloalkyl mit 3 bis 9 Kohlenstoffatomen steht, wobei als Substituenten Halogen, Alkoxy und Alkylthio mit je 1 bis 4 Kohlenstoffatomen, Alkyl, Alkylcarbonyl und Alkylcarbonyloxy mit 1 bis 5 Kohlenstoffatomen je Alkylteil, Alkoxycarbonyl mit 1 bis 5 Kohlenstoffatomen im Alkoxyteil, und Halogenalkyl mit 1 bis 3 Kohlenstoffatomen und 1 bis 5 gleichen oder verschiedenen Halogenatomen genannt seien; für gegebenenfalls ein- bis fünffach, gleich oder verschieden substituiertes Cycloalkenyl mit 3 bis 8 Kohlenstoffatomen steht, wobei als Substituenten Halogen, Alkyl und Alkoxy mit je 1 bis 4 Kohlenstoffatomen und Halogenalkyl mit 1 bis 3 Kohlenstoffatomen und 1 bis 5 gleichen oder verschiedenen Halogenatomen genannt seien;

für gegebenenfalls ein- bis fünffach, gleich oder verschieden substituiertes Aryl mit 6 bis 14 Kohlenstoffatomen oder Aralkyl mit 1 bis 3 Kohlenstoffatomen im Alkylteil und 6 bis 14 Kohlenstoffatomen im Arylteil steht, wobei als Substituenten im Arylteil Halogen, Phenyl, Nitro, Cyano, Isocyanato, Hydroxy, Mercapto, Alkyl mit 1 bis 4 Kohlenstoffatomen, Alkoxy und Alkoxycarbonyl mit 1 bis 5 Kohlenstoffatomen je Alkoxyteil, Alkylcarbonyl und Alkylcarbonyloxy mit je 1 bis 5 Kohlenstoffatomen je Alkylteil, Alkylthio mit 1 bis 5 Kohlenstoffatomen, Halogenalkyl, Halogenalkoxy und Halogenalkylthio mit 1 bis 3 Kohlenstoffatomen und 1 bis 5 gleichen oder verschiedenen Halogenatomen je Halogenalkylrest genannt seien; für einen gegebenenfalls einfach bis fünffach, gleich oder verschieden substituierten 5- oder 6-gliedrigen Heterocyclus mit 1 bis 3 Sauerstoff-, Schwefel- und/oder Stickstoffatomen steht, wobei als Substituenten Halogen, Alkyl mit 1 bis 4 Kohlenstoffatomen, Halogenalkyl und Halogenalkoxy mit 1 bis 3 Kohlenstoffatomen und 1 bis 5 gleichen oder verschiedenen Halogenatomen, Alkoxy mit 1 bis 6 Kohlenstoffatomen oder 2 Substituenten an einem Kohlenstoffatom gemeinsam einen Alkylenrest mit 4 oder 5 Kohlenstoffatomen bilden können genannt seien;

$R^2$ für ein- bis fünffach, gleich oder verschieden substituiertes Alkyl oder Alkenyl mit bis zu 12 Kohlenstoffatomen steht, wobei als Substituenten Halogen, Nitro, Cyano oder Alkoxy mit 1 bis 5 Kohlenstoffatomen genannt seien; für ein- bis fünffach, gleich oder verschieden substituiertes Cycloalkyl oder Cycloalkenyl je mit 3 bis 8 Kohlenstoffatomen steht, wobei als Substituenten Halogen, Alkyl und Alkoxy mit je 1 bis 4 Kohlenstoffatomen und Halogenalkyl mit 1 bis 3 Kohlenstoffatomen und 1 bis 5 gleichen oder verschiedenen Halogenatomen genannt seien; ferner für gegebenenfalls ein- bis fünffach, gleich oder verschieden substituiertes Fluorenyl oder Aralkyl mit 6 bis 14 Kohlenstoffatomen im Arylteil und 1 bis 3 Kohlenstoffatomen im Alkylteil steht, wobei als Substituenten im Arylteil Phenyl, Halogen, Nitro, Cyano, Alkyl oder Alkoxy mit je 1 bis 5 Kohlenstoffatomen genannt seien, und

X für Sauerstoff oder Schwefel steht.

3. 3,6-Disubstituierte 1,3,5-Thiadiazin-2-thioxo-4-one der Formel (I) gemäß Anspruch 1, in welcher

$R^1$ für Wasserstoff; für geradkettiges oder verzweigtes Alkyl mit 1 bis 12 Kohlenstoffatomen;

für Alkenyl mit 2 bis 6 Kohlenstoffatomen; für einfach durch Nitro, Cyano, Isocyanato, Fluor, Chlor, Brom, Jod, Hydroxy, Alkoxy mit 1 bis 3 Kohlenstoffatomen, Mercapto, Alkylthio mit 1 bis 3 Kohlenstoffatomen, Alkylcarbonyl und Alkylcarbonyloxy mit 1 bis 3 Kohlenstoffatomen im Alkylteil und Alkoxycarbonyl mit 1 bis 3 Kohlenstoffatomen im Alkoxyteil substituiertes Alkyl mit 1 bis 4 Kohlenstoffatomen und Alkenyl mit 2 bis 6 Kohlenstoffatomen; für Alkinyl mit 3 bis 6 Kohlenstoffatomen; für einfach durch Fluor, Chlor, Brom, Jod, Alkoxy oder Alkylthio mit je 1 bis 3 Kohlenstoffatomen, Alkylcarbonyl und Alkylcarbonyloxy mit je 1 bis 3 Kohlenstoffatomen je Alkylteil substituiertes Alkinyl mit 3 oder 4 Kohlenstoffatomen im Alkinylteil; für gegebenenfalls ein- bis dreifach durch Methyl oder Ethyl substituiertes Cyclopentyl, Cyclohexyl, Cyclopentenyl, Cyclohexenyl oder Cycloheptenyl; für gegebenenfalls ein- bis dreifach, gleich oder verschieden durch Fluor, Chlor, Brom, Jod, Phenyl, Nitro, Cyano, Isocyanato, Methyl, Ethyl, n- und iso-Propyl, tert.-Butyl, Mercapto, Methoxy, Ethoxy, Propoxy, Methoxycarbonyl, Ethoxycarbonyl, Methylcarbonyl, Ethylcarbonyl, Propylcarbonyl, Trichlormethyl, Fluordichlormethyl, Trichlorethyl, Trichlormethoxy und Trichlorethoxy substituiertes Phenyl, Naphthyl, Benzyl, Phenylethyl, Naphthylethyl und Naphthylmethyl; für gegebenenfalls

ein- bis dreifach durch Fluor, Chlor, Methyl, Ethyl oder Propyl substituiertes 1,3-Dioxacyclohexyl, Furyl, Pyrrolyl, Imidazolyl, Piperazinyl, Isoxazolyl, Morpholinyl und 1,5-Dioxaspiroundecanyl steht;

$R^2$ für Alkyl mit 1 bis 5 Kohlenstoffatomen; für einfach durch Fluor, Chlor, Brom, Cyano, Alkoxy mit 1 bis 3 Kohlenstoffatomen substituiertes Alkyl und Alkenyl mit bis zu 6 Kohlenstoffatomen je Rest; für gegebenenfalls durch Alkyl mit 1 bis 3 Kohlenstoffatomen substituiertes Cycloalkyl und Cycloalkenyl mit 4 bis 7 Kohlenstoffatomen und für gegebenenfalls ein- bis dreifach im Arylteil durch Phenyl, Fluor, Chlor, Brom, Nitro, Alkyl und Alkoxy mit je 1 bis 3 Kohlenstoffatomen substituiertes Benzyl und Fluorenyl steht und

X für Sauerstoff oder Schwefel steht.

4. Verfahren zur Herstellung von 3,6-disubstituiertem 1,3,5-Thiadiazin-2-thioxo-4-onen der Formel (I)

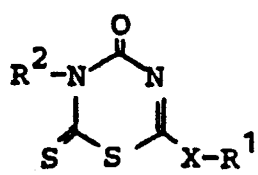

(I)

in welcher

$R^1$ für Wasserstoff; für gegebenenfalls einfach bis mehrfach, gleich oder verschieden substituiertes Alkyl; für gegebenenfalls einfach bis mehrfach, gleich oder verschieden substituiertes Alkenyl; für gegebenenfalls einfach bis mehrfach substituiertes Alkinyl; für gegebenenfalls einfach bis mehrfach, gleich oder verschieden substituiertes Cycloalkyl oder Cycloalkenyl; für gegebenenfalls einfach bis mehrfach, gleich oder verschieden substituiertes Aralkyl oder Aryl oder für einen einfach oder mehrfach, gleich oder verschieden substituierten Heterocyclus steht,

$R^2$ für gegebenenfalls einfach bis mehrfach, gleich oder verschieden substituiertes Alkyl oder Alkenyl; für gegebenenfalls einfach bis mehrfach gleich oder verschieden substituiertes Cycloalkyl oder Cycloalkenyl; für ggf. einfach bis mehrfach, gleich oder verschieden substituiertes Fluorenyl oder Aralkyl steht und

X für Sauerstoff oder Schwefel steht, dadurch gekennzeichnet, daß man 1,3,5-Thiadiazine der Formel (II)

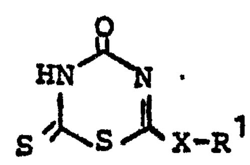

(II)

in welcher

$R^1$ und X die oben angegebene Bedeutung haben,

mit Diazoverbindungen der Formel (III)

$$R^{2'} = N_2 \qquad (III)$$

in welcher

$R^{2'}$ Alkylen, Alkenylen, Cycloalkylen, Cycloalkenylen oder Aralkylen bedeutet, gegebenenfalls in Gegenwart eines inerten Lösungsmittels umsetzt.

5. Schädlingsbekämpfungsmittel, gekennzeichnet durch einen Gehalt an mindestens einem 3,6-disubstituierten 1,3,5-Thiadiazin-2-thioxo-4-on der Formel (I) gemäß Anspruch 1.

6. Verfahren zur Bekämpfung von Schädlingen, dadurch gekennzeichnet, daß man 3,6-disubstituierte 1,3,5-Thiadiazine der Formel (I) gemäß den Ansprüchen 1 und 4 auf Schädlinge und/oder ihren Lebensraum einwirken läßt.

7. Verwendung von 3,6-disubstituierten 1,3,5-Thiadiazinen der Formel (I) gemäß den Ansprüchen 1 und 4 zur Bekämpfung von Schädlingen.

8. Verfahren zur Herstellung von Schädlingsbekämpfungsmitteln, dadurch gekennzeichnet, daß man 3,6-disubstituierte 1,3,5-Thiadiazin-2-thioxo-4-one der Formel (I) gemäß den Ansprüchen 1 und 4 mit Streckmitteln und/oder oberflächenaktiven Mitteln vermischt.